(19) Europäisches Patentamt
European Patent Office
Office européen des brevets

(11) Publication number: 0 584 588 A1

(12) **EUROPEAN PATENT APPLICATION**

(21) Application number: **93112415.0**

(22) Date of filing: **03.08.93**

(51) Int. Cl.5: **A61K 47/02**, A61K 47/08, A61K 31/415

(30) Priority: **03.08.92 JP 226433/92**

(43) Date of publication of application: **02.03.94 Bulletin 94/09**

(84) Designated Contracting States: **AT BE CH DE DK ES FR GB GR IE IT LI LU NL PT SE**

(71) Applicant: **NIPPON CHEMIPHAR CO., LTD.**
**2-2-3, Iwamoto-cho**
**Chiyoda-ku Tokyo(JP)**

(72) Inventor: **Nomura, Yutaka**
**832-113, Mitsubori**
**Noda-shi, Chiba(JP)**
Inventor: **Saito, Katsumi**
**4-17-3, Eki-higashi,**
**Kokubunji-machi**
**Shimotsuga-gun, Tochigi(JP)**

(74) Representative: **Kraus, Walter, Dr. et al**
**Patentanwälte Kraus, Weisert & Partner**
**Thomas-Wimmer-Ring 15**
**D-80539 München (DE)**

(54) **Anti-ulcer composition containing imidazole derivative.**

(57) Disclosed is an anti-ulcer composition to be dissolved in stomach to become directly into contact with walls of the stomach which comprises 100 weight parts of an imidazole derivative of the formula:

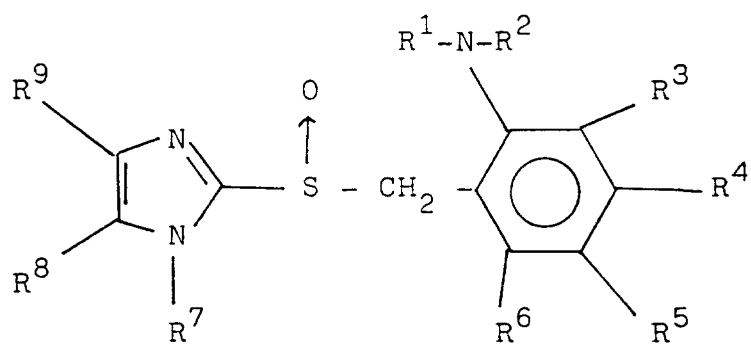

wherein each of $R^1$ and $R^2$ is hydrogen, substituted or unsubstituted alkyl, alkoxyalkyl, cycloalkyl, aryl, aralkyl, or $R^1$ and $R^2$ are combined to form hetero ring; each of $R^3$, $R^4$, $R^5$ and $R^6$ is hydrogen, halogen, substituted or unsubstituted alkoxy, substituted or unsubstituted alkyl, aryl, aryloxy, alkoxycarbonyl, nitro, amino, acyl, or $R^3$ is combined with $R^2$ to form heteroring; $R^7$ is hydrogen, substituted or unsubstituted alkyl, substituted or unsubstituted aryl, substituted or unsubstituted arylcarbonyl, or S-containing heteroring; and each of $R^8$ and $R^9$ is hydrogen, halogen, substituted or unsubstituted alkoxy, unsubstituted or substituted alkyl, alkoxycarbonyl, aralkyl, nitro, amino, acyl, substituted or unsubstituted aryl, or $R^8$ and $R^9$ are combined to form alkylene, and 50 - 2,000 weigt parts of a basic material.

EP 0 584 588 A1

## BACKGROUND OF THE INVENTION

### Field of Invention

The present invention relates to an anti-ulcer composition containing an imidazole derivative, said composition being dissolved in stomach to become directly into contact with walls of the stomach.

### Description of Prior Art

In recent years, considerable interest has been paid to gastric $H^+/K^+$-ATPase as the proton pump in parietal cells. This enzyme is responsible for the secretion of acid into the gastric lumen, and thus is regarded as an important target for peptic ulcer therapy. Since the discovery of the enzyme inhibitor, 5-methoxy-2-[[(4-methoxy-3,5-dimethyl-2-pyridylmethyl]sulfinyl]benzimidazole, namely "omeprazole", there have been reported many analogues having benzimidazole and pyridine moieties. The omeprazole has been recently launched on market and is now employed as anti-ulcer agent of the proton pump inhibitor type in the form of enteric preparations, such as enteric tablets. It is understood that such proton pump inhibitor type preparations are preferably dissolved in stomach and directly affect walls of the stomach, if the proton pump inhibitor is stable in acidic gastric juice. However, since the known proton pump inhibitor type preparations such as omeprazole and lansoprazole, namely, 2-[[(3- methyl-4-(2,2,2-trifluoroethoxy)-2-pyridinyl]methyl]sulfinyl]-1H-benzimidazole, are very rapidly decomposed and inactivated in strong acidic conditions such as pH 1.2 (acidic condition equal to that of the gastric juice of human beings), these are prepared in the form of enteric preprations with enteric coating.

It has been also reported that a benzimidazole type compound having a substituted or unsubstituted aniline moiety in place of the pyridine is employable as an anti-ulcer agent of the proton pump inhibitor type. However, this benzimidazole compound is observed to show relatively poor stability in its storage period. In order to improve the storage stability of the benzimidazole compound having the aniline moiety, it has been proposed to keep the compound in contact with 5 weight % or more of a basic material (EP-A-248,634).

It has been further reported in Chem. Pharm. Bull., 39(7), pp. 1746 (1991), Chem. Pharm. Bull., 40(3), pp. 675-682 (1992), Japanese Patent Provisional Publications No. 2(1990)-138263, No. 3(1991)-163065, No. 1(1989)-131175, and No. 64(1989)-63575, that new imidazole derivatives having the unsubstituted or substituted aniline moiety are effective as the proton pump inhibitor. The Chem. Pharm. Bull., 39(7), pp. 1746 (1991) and Chem. Pharm. Bull., 40(3), pp. 675-682 (1992) indicate that these new imidazole compounds show poor stability in such strong acidic conditions.

## SUMMARY OF THE INVENTION

An object of the present invention is to provide a new anti-ulcer composition of the proton-pump inhibitor type which contains an imidazole derivative as active ingredient and is dissolved in stomach to become into direct contact to walls of the stomach, that is, not the enteric type preparations.

The present invention resides in an anti-ulcer composition to be dissolved in stomach to affect its walls which comprises 100 weight parts of an imidazole derivative having the formula:

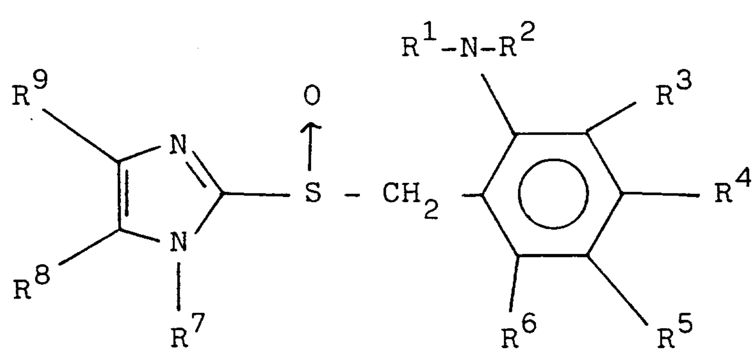

wherein:

each of $R^1$ and $R^2$ independently is hydrogen atom, an alkyl group having 1-8 carbon atoms, an alkyl group of 2-6 carbon atoms having an alkoxy group of 1-4 carbon atoms, a cycloalkyl group having 5-8 carbon atoms, an aryl group having 6-12 carbon atoms, an aralkyl group having 1-4 carbon atoms in its alkyl moiety and 6-12 carbon atoms in its aryl moiety, or an alkyl group of 1-8 carbon atoms having 1-3 halogen atoms, or $R^1$ and $R^2$ are combined to form, together with nitrogen atom to which $R^1$ and $R^2$ are attached, one of 5-8 membered heterocyclic rings;

each of $R^3$, $R^4$, $R^5$ and $R^6$ independently is hydrogen atom, a halogen atom, an alkoxy group having 1-6 carbon atoms, an alkyl group having 1-6 carbon atoms, an aralkyl group having 1-4 carbon atoms in its alkyl moiety and 6-12 carbon atoms in its aryl moiety, an aralkyloxy group having 1-4 carbon atoms in its alkoxy moiety and 6-12 carbon atoms in its aryl moiety, an alkoxycarbonyl group having 2-7 carbon atoms, nitro group, amino group, an acyl group having 1-7 carbon atoms, an alkyl group of 1-6 carbon atoms having 1-3 halogen atoms, or an alkoxy group of 1-6 carbon atoms having 1-3 halogen atoms, or $R^3$ is combined with $R^2$ to form, together with nitrogen atom to which $R^2$ is attached and two carbon atoms of benzene ring to which $R^3$ is attached, one of 5-8 membered heterocyclic rings;

$R^7$ is hydrogen atom, an alkyl group having 1-6 carbon atoms, an alkyl group of 1-6 carbon atoms which has at least one substituent selected from the group consisting of an aryl group of 4-12 carbon atoms, hydroxyl group, and a halogen atom, an aryl group having 4-12 carbon atoms, an aryl group of 4-12 carbon atoms which has at least one substituent selected from the group consisting of an alkyl group of 1-6 carbon atoms, an alkoxy group of 1-6 carbon atoms, and a halogen atom, an arylcarbonyl group having 7-13 carbon atoms, an arylcarbonyl group of 7-13 carbon atoms which has at least one substituent selected from the group consisting of an alkyl group of 1-6 carbon atoms, an alkoxy group of 1-6 carbon atoms, and a halogen atom, or a 5-8 membered heterocyclic group containing a sulfur atom as its ring member; and

each of $R^8$ and $R^9$ independently is hydrogen atom, a halogen atom, an alkoxy group having 1-6 carbon atoms, an alkyl group having 1-6 carbon atoms, an alkoxycarbonyl group having 2-7 carbon atoms, an aralkyl group having 1-4 carbon atoms in its alkyl moiety and 6-12 carbon atoms in its aryl moiety, nitro group, amino group, an acyl group having 1-7 carbon atoms, an alkyl group of 1-6 carbon atoms having 1-3 halogen atoms, an alkoxy group of 1-6 carbon atoms having 1-3 halogen atoms, an aryl group having 6-12 carbon atoms, or an aryl group of 6-12 carbon atoms which has at least one substitutent selected from the group consisting of an alkyl group of 1-6 carbon atoms, an alkoxy group of 1-6 carbon atoms and a halogen atom, or $R^8$ and $R^9$ are combined to form an alkylene chain of 3-5 carbon atoms,

and

50 to 2,000 (preferably, 110 to 1,000, more preferably 210 to 500) weight parts of a basic material.

The anti-ulcer compositions of the present invention are employable for treatment of mammals such as human beings and domestic animals such as dogs, cats, horses, who suffer from stomach ulcer, by way of direct contact with walls of their stomachs.

## DETAILED DESCRIPTION OF THE INVENTION

The imidazole derivatives of the formula (I) per se are known and can be prepared in the manner described in Chem. Pharm. Bull., 39(7), pp. 1746 (1991), Chem. Pharm. Bull., 40(3), pp. 675-682 (1992), Japanese Patent Provisional Publications No. 2(1990)-138263, No. 3(1991)-163065, No. 1(1989)-131175, and No. 64(1989)-63575. It is known that these imidazole derivatives show excellent antiulcer actions.

Details of the groups and atoms mentioned for the formula (1) are described below.

$R^1$ and $R^2$ are the same or different from each other and each represents is hydrogen atom; an alkyl group having 1-8 carbon atoms such as methyl, ethyl, propyl, isopropyl, butyl, isobutyl, pentyl, neopentyl, hexyl, heptyl, octyl or 2-ethylhexyl; an alkyl group of 2-6 carbon atoms having an alkoxy group of 1-4 carbon atoms such as methoxyethyl, methoxypropyl, methoxybutyl, methoxypentyl, ethoxyethyl, ethoxypropyl, ethoxybutyl, propoxyethyl, propoxypropyl or butoxyethyl; a cycloalkyl group having 5-8 carbon atoms such as cyclopentyl, cyclohexyl, cycloheptyl, or cyclooctyl; an aryl group having 6-12 carbon atoms such as phenyl, naphthyl, tolyl, or xylyl; an aralkyl group which has 6-12 carbon atoms in its aryl moiety and 1-4 carbon atoms in its alkyl moiety such as benzyl, phenylethyl, naphthylmethyl or tolylmethyl; an alkyl group of 1-8 carbon atoms having 1 to 3 halogen atoms such as chloromethyl, chloroethyl, bromomethyl, fluoromethyl, fluoroethyl, fluoropropyl, fluorobutyl, difluoromethyl, trifluoromethyl, or trifluoroethyl. Otherwise, $R^1$ and $R^2$ are combined to form, in conjunction with the nitrogen atom to which $R^1$ and $R^2$ are attached, one of 5-8 membered heterocyclic rings such as pyrrole ring, pyrroline ring, pyrrolidine ring, morphorine ring, pyridine ring, piperidine ring, or perhydroazepine ring.

$R^3$, $R^4$, $R^5$ and $R^6$ are, all or in part, the same or different from each other, and each represents hydrogen atom; a halogen atom such as fluorine, chlorine, or bromine; an alkoxy group having 1-6 carbon atoms such as methoxy, ethoxy, propoxy, isopropoxy, butoxy, isobutoxy, tert-butoxy, pentoxy or hexyloxy; an alkyl group having 1-6 carbon atoms such as methyl, ethyl, propyl, isopropyl, butyl, isobutyl, tert-butyl, pentyl or hexylyl; an aralkyl group which has 6-12 carbon atoms in its aryl moiety and 1-4 carbon atoms in its alkyl moiety such as benzyl, phenylethyl, naphthylmethyl or tolylmethyl; an aralkyloxy group which has 6-12 carbon atoms in its aryl moiety and 1-4 carbon atoms in its alkoxy moiety such as benzyloxy, phenylethyloxy, naphthylmethyloxy or tolylmethyloxy; an alkoxycarbonyl having 2-7 carbon atoms such as methoxycarbonyl, ethoxycarbonyl, propoxycarbonyl, isopropoxycarbonyl, butoxycarbonyl, pentoxycarbonyl or hexyloxycarbonyl; nitro group; amino group; an acyl having 1-7 carbon atoms such as formyl, acetyl, propionyl, isopropionyl, butyryl, isobutyryl, valeryl, or isovaleryl; an alkyl group of 1-6 carbon atoms which has 1 to 3 halogen atoms such as fluoromethyl, chloromethyl, bromomethyl, difluoromethyl, trifluoromethyl, fluoroethyl, trifluoroethyl, fluoropropyl, fluorobutyl, fluoropentyl, or fluorohexyl; or an alkoxy group of 1-6 carbon atoms which has 1 to 3 halogen atoms such as flouromethoxy, chloromethoxy, bromomethoxy, difluoromethoxy, trifluoromethoxy, fluoroethoxy, fluoropropoxy, fluoroisopropoxy, fluorobutoxy, fluoropentoxy, or fluorohexyloxy. Otherwise, $R^3$ is combined with $R^2$ to form, together with the nitrogen atom to which $R^2$ is attached and the carbon atom of the phenyl group to which $R^3$ is attached, one of 5- to 8-membered rings such as pyrrolidine or piperidine.

$R^7$ is a hydrogen atom; an alkyl group of 1-6 carbon atoms which may be substituted with one or more aryl groups of 4-12 carbon atoms, hydroxyl groups, or halogen atoms such as methyl, ethyl, propyl, isopropyl, butyl, isobutyl, t-butyl, pentyl, benzyl, phenylethyl, phenylpropyl, tolylmethyl, xylylmethyl, naphthylmethyl, naphthylethyl, hydroxymethyl, hydroxyethyl, hydroxypropyl, hydroxybutyl, fluoromethyl, 2-pyridiylemthyl, 2-(2-pyridyl)ethyl, fluoroethyl, fluoropropyl, trifluoromethyl, chloromethyl, or bromomethyl; an aryl group of 4-12 carbon atoms which may be substituted with an alkyl group of 1-6 carbon atoms, an alkoxy group of 1-6 carbon atoms, or a halogen atom, such as phenyl, naphthyl, tolyl, xylyl, methoxyphenyl, ethoxyphenyl, dimethoxyphenyl, chlorophenyl, fluorophenyl, bromophenyl, 3-pyridyl, 2-pyridyl, 3-methylpyridyl, or 5-trifluoromethyl-2-pyridyl; an arylcarbonyl of 7-13 carbon atoms which may be substituted with an alkyl group of 1-6 carbon atoms, an alkoxy group of 1-6 carbon atoms, or a halogen atom, such as benzoyl, toluoyl, methoxybenzoyl, ethoxybenzoyl, or fluorobenzoyl; or a heterocyclic group of 5-8 members having a sulfur atom as its ring member, such as thiophene or tetrahydrothiophene.

$R^8$ and $R^9$ are the same or different from each other, and each represents hydrogen atom; a halogen atom such as fluorine, chlorine, bromine or iodine; an alkoxy group having 1-6 carbon atoms such as methoxy, ethoxy, propoxy, butoxy, isobutoxy, pentoxy, or hexyloxy; an alkyl group having 1-6 carbon atoms such as methyl, ethyl, propyl, isopropyl, butyl, isobutyl, pentyl or hexyl; an alkoxycarbonyl group having 2-7 carbon atoms, such as methoxycarbonyl, ethoxycarbonyl, propoxycarbonyl, butoxycarbonyl, isobutoxycarbonyl, pentoxycarbonyl, or hexyloxycarbonyl; nitro group; amino group; an acyl group having 1-7 carbon atoms such as formyl, acetyl, propionyl, butyryl, isobutyryl, valeryl or isovaleryl; an alkyl group of 1-6 carbon atoms having 1 to 3 halogen atoms, such as fluoromethyl, chloromethyl, bromomethyl, difluoromethyl, trifluoromethyl, fluoroethyl, trifluoroethyl, fluoropropyl, fluorobutyl, fluoropentyl or fluorohexyl; an alkoxy group of 1-6 carbon atoms having 1 to 3 halogen atoms, such as flouromethoxy, chloromethoxy, bromomethoxy, difluoromethoxy, trifluoromethoxy, fluoroethoxy, fluoropropoxy, fluorobutoxy, fluoropentoxy or fluorohexyloxy; or an aryl group (e.g., phenyl and naphthyl) which may have at least one substitutent (generally one, two or three substituents) selected from the group consisting of an alkyl group of 1-6 carbon atoms (e.g., methyl, ethyl, propyl, isopropyl, butyl, or isobutyl), an alkoxy group of 1-6 carbon atoms (e.g., methoxy, ethoxy, propoxy, isopropoxy, butoxy, or isobutoxy) and a halogen atom (e.g., fluorine, chlorine, or bromine), such as phenyl, naphthyl, tolyl, xylyl, methoxyphenyl, ethoxyphenyl, dimethoxyphenyl, chlorophenyl, fluorophenyl, or bromophenyl. Otherwise, $R^8$ and $R^9$ are combined to form an alkylene chain of 3-5 carbon atoms. In other words, $R^8$ and $R^9$ are combined to form, together with two carbon atoms of the imidazole ring to which $R^8$ and $R^9$ are attached, one of 5-7 membered alicyclic rings such as cyclopentenyl, cyclohexenyl, methylcyclohexenyl, dimethylcyclohexenyl or cycloheptenyl.

The following groups are preferably adopted for the imidazole derivative of the formula (1).

1) Each of $R^1$ and $R^2$ independently is hydrogen atom, an alkyl group of 1-8 carbon atoms, or an alkyl group of 2-6 carbon atoms having an alkoxy group of 1-4 carbon atoms.

2) Each of $R^3$, $R^4$ and $R^6$ is hydrogen atom, and $R^5$ is hydrogen atom or an alkoxy group of 1-6 carbon atoms.

3) $R^7$ is hydrogen atom.

4) each of $R^8$ and $R^9$ of the formula is a hydrogen atom.

Examples of the imidazole derivatives accroding to the formula (1) are listed below:
2-[5-methoxy-(2-methoxyethylamino)benzylsulfinyl]imidazole,
2-[2-(2-methoxyethylamino)benzylsulfinyl]imidazole,
2-[2-(2-isopropoxyethylamino)benzylsulfinyl]imidazole,
2-[2-(2-methoxyethylamino)-5-methylbenzylsulfinyl]imidazole,
2-[2-(2-methoxyethylamino)-6-methylbenzylsulfinyl]imidazole,
2-[2-(2-ethoxyethylamino)benzylsulfinyl]imidazole,
2-[2-(3-methoxypropylamino)benzylsulfinyl]imidazole,
2-[2-(2-ethoxyethylamino)-5-methylbenzylsulfinyl]imidazole,
2-[4,6-dimethyl-5-methoxy-2-(2-methoxyethylamino)benzylsulfinyl]imidazole,
2-(2-methylaminobenzylsulfinyl)-4,5,6,7-tetrahydro-1H-benzimidazole,
2-(2-dimethylaminobenzylsulfinyl)-4,5,6,7-tetrahydro-1H-benzimidazole,
2-(2-aminobenzylsulfinyl-4,5,6,7-tetrahydro-1H-benzimidazole,
2-(2-methylaminobenzylsulfinyl)imidazole,
2-[(2-isobutylamino)benzylsulfinyl]imidazole,
2-[(2-dimethylamino)benzylsulfinyl]imidazole,
2-[(5-methyl-2-methylamino)benzylsulfinyl]imidazole,
2-(2-aminobenzylsulfinyl)imiazole,
2-[(2-methylamino)benzylsulfinyl]imidazole,
2-[(2-isobutylamino-5-nitro)benzylsulfinyl]imidazole,
2-[(4-chloro-2-isobutylamino)benzylsulfinyl]imidazole,
2-[(2-isopropylamino)benzylsulfinyl]imidazole,
2-[(2-ethylamino)benzylsulfinyl]imidazole,
2-[(2-benzylamino)benzylsulfinyl]imidazole,
2-[(2-benzylamino)benzylsulfinyl]imidazole,
2-[(2-isobutylamino-5-methoxy)benzylsulfinyl]imidazole,
2-[(2,3-dimethoxy-6-isobutylamino)benzylsulfinyl]imidazole,
2-[(2-methyl-6-methylamino)benzylsulfinyl]imidazole,
2-[(2-isobutylamino-5-trifluoromethoxy)benzylsulfinyl]imidazole,
4-methyl-2-[(2-methylamino)benzylsulfinyl]imidazole,
2-[(5-chloro-2-isobutylamino)benzylsulfinyl]imidazole,
2-[(2-isobutylamino-6-methoxy)benzylsulfinyl]imidazole,
2-[(5-fluoro-2-isobutylamino)benzylsulfinyl]imidazole,
2-[(2-isobutylamino-6-methyl)benzylsulfinyl]imidazole,
2-[(2-isobutylamino-4-methyl)benzylsulfinyl]imidazole,
2-[(2-chloro-6-isobutylamino)benzylsulfinyl]imidazole,
2-[(2-isobutylamino-3-methyl)benzylsulfinyl]imidazole,
2-[(2-isobutylamino-3-methoxy)benzylsulfinyl]imidazole,
2-[(3-methyl-2-methylamino)benzylsulfinyl]imidazole,
2-[(2-propylamino)benzylsulfinyl]imidazole,
2-[(2-butylamino)benzylsulfinyl]imidazole,
2-[(2-isobutylamino)benzylsulfinyl]-4,5,6,7-tetrahydro-1H-imidazole,
4-ethyl-5-methyl-2-[(2-methylamino)benzylsulfinyl]imidazole,
2-(2-dimethylaminobenzylsulfinyl)-1-(3-methylpyridin-2-yl)imidazole,
2-[2-(2,2,2-trifluoroethylamino)benzylsulfinyl]imidazole,
2-[8-(1,2,3,4-tetrahydro)quinolyl]methylsulfinylimidazole,
2-(2-dimethylaminobenzylsulfinyl)-1-(2-fluorophenyl)imidazole,
2-(2-dimethylaminobenzylsulfinyl)-1-(2-pyridyl)imidazole,
2-(2-morpholinobenzylsulfinyl)-1-(2-pyridyl)imidazole,
2-(2-cyclohexylaminobenzylsulfinyl)-1-(2-pyridyl)imidazole, and
2-(2-dimethylaminobenzylsulfinyl)-1-(5-methoxy-2-pyridyl)imidazole.

The basic material to be employed with the imidazole derivative of the formula (1) means a material which shows pH 7 or higher, preferably pH 8 or higher, in the form of its aqueous solution or suspension.

The basic material preferably is a hydroxide, an oxide, or a salt with a weak inorganic acid of a metal such as an alkali metal, an alkaline earth metal and aluminum. More concretely, the basic material preferably is a hydroxide such as alumina magnesium hydroxide, aluminum hydroxide or magnesium hydroxide. Examples of the salts with a weak inorganic acid include carbonates such as sodium carbonate, potassium carbonate, calcium carbonate, and magnesium carbonate; hydrogen carbonates such as sodium

hydrogen carbonate, potassium hydrogen carbonate, magnesium hydrogen carbonate, and calcium hydrogen carbonate; phosphates such as potassium monohydrogen phosphate, potassium phosphate and sodium phosphate; silicates such as magnesium silicate, and coprecipitation products of hydroxide with carbonate such as aluminum hydroxide-sodium hydrogen carbonate coprecipitation product, syntesized hydrotalcite, and aluminum hydroxidemagnesium carbonate-calcium carbonate coprecipitation product.

The basic material may be a salt of an organic acid (e.g., carboxylic acid) with an alkali metal, an alkaline earth metal, aluminum, or amine. Examples of the organic acids are acetic acid, propionic acid, tartaric acid, benzoic acid, alginic acid, edetic acid (EDTA), citric acid, glycyrrhizinic acid, glutamic acid, gluconic acid, succinic acid, fumaric acid, salicylic acid, and lactic acid. Examples of the metals, include sodium, potassium, calcium, magnesium, and aluminum. Examples of the amines include isopropanolamine, diphenylamine, ethanolamine, and benzylamine.

The basic material may be a basic amino acid such as histidine or an amine such as diisopropanolamine, diphenylamine, ethanolamine or benzylamine.

The basic material can be used singly or in combination of two or more materials (e.g., a combination of an inorganic basic material and an organic basic material, a combination of two or more inorganic basic materials, and a combination of two or more organic basic materials).

The basic material is employed in an amount of 50 to 2,000 weight parts, preferably 110 to 1,000 weight parts, more preferably 210 to 500 weight parts, per 100 weight parts of the imidazole derivative of the formula (1). If the amount of the basic material per the imidazole derivative is too small, the imidazole derivative does not show enough stability in stomach, that is, in acidic gastric juice. If the amount of the basic material is too large, the administration of the composition is disturbed.

The amount of the basic material can be adjusted depending on the nature of mammals to be treated. If the human being is treated, the amount of the basic material should be relatively large, such as larger than 100 weigt parts, or larger than 210 weight parts, because the acidic condition in the stomach of human being is very severe (such as pH 1.2). However, if the horse or other domestic animals are treated, the amount of the basic material can be decreased, because the acidic condition of the gastric juice in their stomachs are relatively mild such as about pH 2-4.

The anti-uncler composition according to the present invention is administered orally to human beings and other mammals such as domestic animals who are suffering from stomach ulcer. Preparation forms for oral administration may be, for example, tablets (or pellets), capsules, powder, granules, syrup, paste, and the like. For these preparations, excipients, disintegrants, binders, lubricants, pigments, diluents and the like which are commonly employed in the art may be used. The excipients may include dextrose, lactose and the like. Starch, carboxymethylcellulose calcium and the like may be used as the disintegrants. Magnesium stearate, talc and the like may be used as the lubricants. The binders may be hydroxypropylcellulose, gelatin, and polyvinylpyrrolidone.

The dose usually is about 10 mg/day to 500 mg/day in the case of oral administration, for an adult. The dose may be either increased or decreased depending on the age, race, and other conditions of the patients.

For domestic animals such as horse, pig, cow, dog and cat, the dose usually is about 0.2 mg/kg/day to 10 mg/kg/day.

Examples of the present invention are given below.

## EXAMPLE 1

### Stability of Omeprazole and Imidazole Derivative of Formula (1) in Acidic Conditions

The stability of the compound in acidic conditions was determined at 37°C in Britten-Robinson buffer which had been previously adjusted to pH 3.0, 5.0 or 7.0. The compound was dissolved in this buffer by supersonic waves, and the filtered solution was monitored by high performance liquid chromatography (HPLC) (Tosoh TSK-gel ODS-120T 4.6-250mm, $CH_3CN$/10 mM phosphate buffer (pH 6.0), 1 ml/min., room temperature (25°C), detector ultraviolet (UV) 240-250 nm). Half-lives ($T_{1/2}$) were determined from linear regression of the concentration vs. time (h). The results are give below.

| | $(T_{1/2})$ (h) | | |
|---|---|---|---|
| | pH 3.0 | pH 5.0 | pH 7.0 |
| Omeprazole | 0.05 | 0.34 | 27 |
| Imidazole derivative* | 0.03 | 0.93 | 63 |

*) 2-[(2-isobutylamino)benzylsulfinyl]imidazole

EXAMPLE 2

Storage Stability of Imidazole Derivative of Formula (1)

2-[(2-Isobutylamino)benzysulfinyl]imidazole was subjected to storage stability test at 60°C, atmospheric condition, in a dark room, and 40°C, 75%RH in a dark room. The results are given below.

| 60°C | Storage Period | Decomposition | Undecomposed* |
|---|---|---|---|
| | 7 days | None | 99% |
| | 14 days | None | 101% |
| | 21 days | None | 100% |
| | 28 days | None | 100% |
| | 35 days | None | 99% |
| | 56 days | None | 100% |
| | 70 days | None | 100% |
| | 102 days | None | 100% |
| | 133 days | None | 100% |
| | 184 days | None | 99% |

*) Found value

| 40°C, 75%RH | Storage Period | Decomposition | Undecomposed* |
|---|---|---|---|
| | 1 month | None | 101% |
| | 2 months | None | 101% |
| | 3 months | None | 100% |
| | 4 months | None | 100% |
| | 5 months | None | 100% |
| | 6 months | None | 100% |

*) Found value

EXAMPLE 3

Synthesis of 2-[(2-Isobutylamino)benzylsulfinyl]imidazole (Imidazole derivative A)

(i) 2-[(2-Isobutylamino)benzylthio]imidazole

2-Isobutylaminobenzyl chloride hydrochloride (1.0 g) was added to 2-mercaptoimidazole (427 mg) in ethanol (5 ml) at room temperature for approx. 5 min. The resulting homogeneous solution was stirred for one hour at room temperature, and placed under reduced pressure at a temperature of not higher than 40°C to remove ethanol. The residue was extracted with chloroform, after addition of a small amount of water and a saturated aqueous sodium hydrogen carbonate. The separated chloroform portion was dried over sodium sulfate and then placed under reduced pressure to evaporate the solvent, to give 1.05 g (yield: 94%) of the desired product as a white crystalline powder.

IR ($\nu^{KBr}$)$cm^{-1}$: 3390, 2950, 1605, 1515, 1460, 1420, 1315, 1100, 750.

$^1$H-NMR ($CDCl_3$) $\delta$ : 0.88 (d, 6H, J = 7Hz), 1.84 (m, 1H), 2.84 (d, 2H, J = 7Hz), 4.12 (s, 2H), 6.2-7.1 (m, 6H).

(ii) 2-[(2-Isobutylamino)benzylsulfinyl]imidazole

To a mixture of 2-[(2-Isobutylamino)benzylthio]imidazole (1 g) obtained in (i) above, chloroform (40 ml), and methanol (10 ml) was portionwise added under chilling with ice, m-chloroperbenzoic acid (equivalent molar amount). After completion of the reaction was confirmed using TLC, the reaction mixture was made alkaline by addition of saturated aqueous sodium hydrogen carbonate and extracted with chloroform. The chloroform portion was then treated successively with three portions of 10 ml of 0.1 N aqueous NaOH and one portion of 20 ml of 0.1 N aqueous NaOH to transfer the desired product into the aqueous portions. The obtained four aqueous portions were separately made ammonia-alkaline by addition of 20% aqueous ammonium chloride. The precipitated crystals were collected, washed sufficiently with ether, and dried to give 0.6 g (yield: 56.5%) of the desired product as a white crystalline powder.

IR ($\nu^{KBr}$)$cm^{-1}$: 3360, 3340, 3160, 2950, 1600, 1580, 1515, 1468, 1315, 1020, 740.

$^1$H-NMR ($CDCl_3/CD_3OD$ = 1/1, v/v) $\delta$: 1.94 (d, 6H, J = 7Hz), 1.94 (m, 1H), 2.90 (d, 2H, J = 7Hz), 4.32 (d, 1H, J = 13Hz), 4.54 (d, 1H, J = 13Hz), 6.4-7.3 (m, 4H), 7.24 (s, 2H).

m.p.: 132 - 133°C (decomp.)

The cytoprotective action on gastrointestinal tract provided by Imidazole derivative A is shown by the following experimental results obtained in the HCl-ethanol erosion model.

Experimental procedures

SD rats were fested for 24 hours. To the rat was given orally a hydrochloric acid-ethanol mixture (150 mM HCl in 60% ethanol) at 1 ml/200 g (weight). One hour later, each rat was killed by ether and the stomach was examined for any damage (erosion) in the glandular portion. Length (mm) of each erosion was measured, and the total length (mm) of the erosions observed in one rat was calculated.

The compound to be tested was administered orally to the rat 30 min. before the administration of HCl-ethanol mixture.

Results

It was observed that Imidazole derivative A suppressed HCl-ethanol erosion almost proportionally to its dose, and showed significant suppression at a dose of more than 30 mg/kg. $ED_{50}$ was 3.6 mg/kg.

(2) Preparation of anti-ulcer agent to be dissolved in stomach (according to Invention)

| Inventive Preparation (one tablet) | |
|---|---|
| Imidazole derivative A | 40.0 mg |
| Lactose | 45.0 mg |
| Crystalline cellulose | 15.0 mg |
| Hydroxypropylcellulose (L-HPC) | 10.0 mg |
| Alumina magnesium hydroxide | 115.0 mg |
| Hydroxypropylcellulose (HPC-SL) | 3.0 mg |
| Magnesium stearate | 2.0 mg |
| Total | 230.0 mg |

Imidazole derivative A, lactose, crystalline cellulose, hydroxypropylcellulose (L-HPC) and alumina magnesium hydroxide were mixed and stirred. To the stirred mixture was added 5% aqueous hydroxypropylcellulose (HPC-SL). The resulting mixture were kneaded, and processed to give granules. The grunules were dried in a dryer and sieved on 16 mesh sieve. The sieved granules were well mixed with magnesium stearate, and the mixture was processed in a rotary pelletizer to give the desired tablets.

(3) Preparation of anti-ulcer agent to be dissolved in stomach (for Comparison)

| Comparative Preparation (one tablet) | |
|---|---|
| Imidazole derivative A | 40.0 mg |
| Lactose | 48.5 mg |
| Corn starch | 20.0 mg |
| Hydroxypropylcellulose (L-HPC) | 8.0 mg |
| Alumina magnesium hydroxide | 1.0 mg |
| Hydroxypropylcellulose (HPC-SL) | 1.5 mg |
| Magnesium stearate | 1.0 mg |
| Total | 120.0 mg |

The comparative preparation was in the same manner as that employed for the inventive preparation.

(4) Remaining ratio of Imidazole derivative in acidic solutions

The tablets obtained above were dissolved in Solution I (pH = 1.2) defined in Japanese Pharamacopoeia and Solution II (pH = 2.0) to determine the remaining ratio of Imidazole derivative (ratio by wt.% of undecomposed derivative per the tested derivative) of Imidazole derivative A after lapse of the predetermined periods, in the following manner.

According to Japanese Pharmacopoeia, Solution I was prepared by dissolving 2.0 g of sodium chloride in a mixture of hydrochloric acid (7.0 ml) and water to give a solution of 1,000 ml. Solution I was colorless and clear, and had pH approx. 1.2.

Solution II was prepared by dissolving 2.0 g of sodium chloride in 900 ml of water, adding 0.1 N hydrochloric acid to the solution to adjust it to pH 2.0, and then adding water to the soluiton to 1,000 ml.

In a beaker was placed 60 ml of Solution I and warmed to keep 37°C. In the solution was placed one tablet, and the solution was then shaken for the predetermined periods (15 min., 30 min., and 60 min.). To the solution in the beaker was added 60 ml of 0.1 N NaOH-methanol solution, and both were mixed. The resulting mixture was placed in a measuring flask, and then methanol was added to the mixture to adjust the total volume to 500 ml (Sample solution).

Separately, approx. 0.080 g of Imidazole derivative A was taken and its weight was accurately measured. The measured sample was dissolved in methanol to give precisely 250 ml of Standard solution.

5 Microlitters of each of Sample solution and Standard solution were taken and subjected separately to liquid chromatography. From the peak area in the chart obtained by the liquid chromatography, the amount

of Imidazole derivative A remaining in Sample solution was determined.

The liquid chromatography was performed under the following conditions:

| | |
|---|---|
| Detector: | UV absorption spectrophotometer (measured at 265 nm) |
| Column: | ODS (YMC pack A-302, 4.6x150 mm) |
| Column Temperature: | room temperature |
| Eluent: | acetonitrile/pH 7.0 phosphate buffer (5:4) |
| Flow rate: | 1.1 ml/min. |

The above-described procedure was repeated except for using Solution II in place of Solution I to determine the amount remainining in Solution II after the predetermined periods.

Disintegration period of the tablet was examined on the inventive tablet and comparative tablet according to Disintegration Test defined in Japanese Pharmacopoeia. The test was repeated twice using 6 tablets for each of the inventive tablet and comparative tablet. The mean disintegration periods were 11 min. for Inventive tablet and 5 min. for Comparative tablet. Thus, no significant effect was given to the remaining ratio by the disintegration of tablet.

The remaining ratios obtained in the above measurements are set forth in Table 1.

Table 1

| Acidic Solution | Tablet | Remaining Ratio | | |
|---|---|---|---|---|
| | | 15 min. | 30 min. | 60 min. |
| Solution I | Inventive<br>Comparative | 63%<br>15% | 38%<br>2% | 32%<br>0% |
| Solution II | Inventive<br>Comparative | 93%<br>52% | 86%<br>29% | 84%<br>14% |

From the experimental data in Table 1, it is clear that Imidazole derivative A in the comparative tablet remains only 15% after 15 min., and almost no Imidazole derivative A remains after 30 min., while Imidazole derivative A in the inventive tablet remains 63% after 15 min., and 32% after 60 min. Accordingly, it is understood that the inventive tablet keeps Imidazole derivative A prominently stably in the acidic conditions, as compared with the comparative tablet does. Even in Solution II, most of Imidazole derivative A is decomposed after 60 min. in the comparative tablet, while the inventive tablet keeps Imidazole derivative A stably even after 60 min. These results show superiority of the tablet according to the present invention.

## EXAMPLE 4

### Synthesis of 2-[4,6-dimethyl-2-(2-ethoxyethylamino)-5-methoxybenzylsulfinyl]imidazole (Imidazole derivative B)

In the same manner as that for the synthesis of Imidazole derivative A, Imidazole derivative B was synthesized.

IR ($\nu^{KBr}$)$cm^{-1}$: 3350, 3140, 2900, 2850, 1610, 1580, 1500, 1455, 1410, 1340, 1240, 1120, 1095, 1020, 1005, 960, 860, 820, 780.

$^1$H-NMR ($CDCl_3/CD_3OD$ = 1/1, v/v) $\delta$: 1.24 (t, 3H, J = 8Hz), 2.15 (s, 3H), 2.26 (s, 3H) 3.30 (t, 2H, J = 5Hz), 3.62 (s, 3H), 3.4-3.8 (m, 4H), 4.35 (d, 1H, J = 14Hz), 4.60 (d, 1H, J = 14Hz), 6.46 (s, 1H), 7.25 (s, 2H).

m.p.: 117 - 118°C (decomp.)

The inventive tablet was prepared in the same manner as that of Example 3 except for using Imidazole derivative B. The prepared tablet was subjected to the test for determining the remaining ratio of Imidazole derivative in acidic solutions as described in Example 3. It was found that the inventive tablet containing Imidazole derivative B gave high remaining ratios (similar to the ratios obtained in Example 3) in Solutions I and II.

EXAMPLE 5

Synthesis of 2-[2-(2-ethoxyethylamino)-5-methoxybenzylsulfinyl]imidazole (Imidazole derivative C)

In the same manner as that for the synthesis of Imidazole derivative A, Imidazole derivative C was synthesized.

IR ($\nu^{KBr}$)$cm^{-1}$: 3350, 2960, 2850, 2750, 1515, 1440, 1420, 1290, 1230, 1205, 1110, 1030, 960, 800, 765, 495.

$^1$H-NMR ($CDCl_3/CD_3OD$ = 1/1, v/v) $\delta$: 1.22 (t, 3H, J = 7Hz), 3.19 (m, 2H), 3.5-3.8 (m, 4H), 3.61 (s, 3H), 4.22 (d, 1H, J = 14Hz), 4.57 (d, 1H, J = 14Hz), 6.34 (d, 1H, J = 3Hz), 6.62 (d, 1H, J = 9Hz), 6.75 (dd, 1H, J = 3Hz, 9Hz), 7.16 (s, 2H), 11.32 (bs, 1H).

m.p.: 120.5 - 122°C (decomp.)

The inventive tablet was prepared in the same manner as that of Example 3 except for using Imidazole derivative C. The prepared tablet was subjected to the test for determining the remaining ratio of Imidazole derivative in acidic solutions as described in Example 3. It was found that the inventive tablet containing Imidazole derivative C gave high remaining ratios (similar to the ratios obtained in Example 3) in Solutions I and II.

EXAMPLE 6

Synthesis of 2-[2-(2-ethoxyethylamino)-4-methylbenzylsulfinyl]imidazole (Imidazole derivative D)

In the same manner as that for the synthesis of Imidazole derivative A, Imidazole derivative D was synthesized.

IR ($\nu^{KBr}$)$cm^{-1}$: 3390, 2970, 2900, 1610, 1580, 1530, 1310, 1300, 1115, 1100, 1000, 940, 885.

$^1$H-NMR ($CDCl_3/CD_3OD$ = 1/1, v/v) $\delta$: 1.25 (t, 3H, J = 7Hz), 2.27 (s, 3H), 3.56 (t, 2H, J = 5Hz), 3.59 (q, 2H, J = 7Hz), 3.69 (t, 2H, J = 5Hz), 4.29 (d, 1H, J = 14Hz), 4.53 (d, 1H, J = 14Hz), 6.3-6.7 (m, 3H), 7.22 (s, 2H).

m.p.: 139 - 142°C (decomp.)

The inventive tablet was prepared in the same manner as that of Example 3 except for using Imidazole derivative D. The prepared tablet was subjected to the test for determining the remaining ratio of Imidazole derivative in acidic solutions as described in Example 3. It was found that the inventive tablet containing Imidazole derivative d gave high remaining ratios (similar to the ratios obtained in Example 3) in Solutions I and II.

EXAMPLE 7

Synthesis of 2-[2-(2-ethoxyethylamino)-6-methylbenzylsulfinyl]imidazole (Imidazole derivative E)

In the same manner as that for the synthesis of Imidazole derivative A, Imidazole derivative E was synthesized.

IR ($\nu^{KBr}$)$cm^{-1}$: 3360, 2970, 2860, 1585, 1520, 1480, 1435, 1320, 1125, 1105, 1095, 1020, 770, 750.

$^1$H-NMR ($CDCl_3/CD_3OD$ = 1/1, v/v) $\delta$: 1.23 (t, 3H, J = 7Hz), 2.22 (s, 3H), 3.26 (6, 2H, J = 5Hz), 3.58 (q, 2H, J = 7Hz), 3.71 (t, 2H, J = 5Hz), 4.39 (d, 1H, J = 14Hz), 4.63 (d, 1H, J = 14Hz), 6.4-7.2 (m, 3H), 7.27 (s, 2H).

m.p.: 101 - 102°C (decomp.)

The inventive tablet was prepared in the same manner as that of Example 3 except for using Imidazole derivative E. The prepared tablet was subjected to the test for determining the remaining ratio of Imidazole derivative in acidic solutions as described in Example 3. It was found that the inventive tablet containing Imidazole derivative E gave high remaining ratios (similar to the ratios obtained in Example 3) in Solutions I and II.

EXAMPLE 8

Synthesis of 2-[5-methoxy-2-(2-methoxyethylamino)benzylsulfinyl]imidazole (Imidazole derivative F)

In the same manner as that for the synthesis of Imidazole derivative A, Imidazole derivative F was synthesized.

IR ($\nu^{KBr}$)$cm^{-1}$) : 3360, 3330, 2890, 1515, 1290, 1240, 1215, 1110, 1100, 1015, 950.

$^1$H-NMR ($CDCl_3$) $\delta$: 3.20 (m, 3H), 3.38 (s, 3H), 3.61 (s, 3H) 3.60 (m, 2H), 4.22 (d, 1H, J=14Hz), 4.57 (d, 1H, J=14Hz), 6.35 (d, 1H, J=3Hz), 6.62 (d, 1H, J=9Hz), 6.76 (dd, 1H, J=3Hz, 9Hz), 7.17 (s, 2H).

m.p.: 123.5 - 124.5°C (decomp.)

The inventive tablet was prepared in the same manner as that of Example 3 except for using Imidazole derivative F. The prepared tablet was subjected to the test for determining the remaining ratio of Imidazole derivative in acidic solutions as described in Example 3. It was found that the inventive tablet containing Imidazole derivative F gave high remaining ratios (similar to the ratios obtained in Example 3) in Solutions I and II.

EXAMPLE 9

Synthesis of 2-[5-methoxy-2-(2-methoxyethylamino)-6-methylbenzylsulfinyl]imidazole (Imidazole derivative G)

In the same manner as that for the synthesis of Imidazole derivative A, Imidazole derivative G was synthesized.

IR ($\nu^{KBr}$)$cm^{-1}$ : 3350, 2900, 1480, 1460, 1260, 1260, 1130, 1100, 1020, 795, 500.

$^1$H-NMR ($CDCl_3$/$CD_3OD$=1/1, v/v) $\delta$ : 2.13 (s, 3H), 3.1-3.3 (m, 2H), 3.42 (s, 3H), 3.76 (s, 3H), 3.68 (t, 2H, J=5Hz), 4.35 (d, 1H, J=14Hz), 4.65 (d, 1H, J=14Hz), 6.60 (d, 1H, J=8Hz), 6.85 (d, 1H, J=8Hz), 7.25 (s, 2H).

m.p.: 110 - 111°C (decomp.)

The inventive tablet was prepared in the same manner as that of Example 3 except for using Imidazole derivative G. The prepared tablet was subjected to the test for determining the remaining ratio of Imidazole derivative in acidic solutions as described in Example 3. It was found that the inventive tablet containing Imidazole derivative G gave high remaining ratios (similar to the ratios obtained in Example 3) in Solutions I and II.

EXAMPLE 10

Synthesis of 2-[5-methoxy-2-(2-methoxyethylamino)-4-methylbenzylsulfinyl]imidazole (Imidazole derivative H)

In the same manner as that for the synthesis of Imidazole derivative A, Imidazole derivative H was synthesized.

IR ($\nu^{KBr}$)$cm^{-1}$ : 3400, 2900, 1520, 1500, 1465, 1410, 1230, 1110, 1095, 1000, 900, 790, 500.

$^1$H-NMR ($CDCl_3$/$CD_3OD$=1/1, v/v) $\delta$: 2.15 (s, 3H), 3.27 (t, 2H, J=5Hz), 3.43 (s, 3H) 3.62 (s, 3H), 3.62 (t, 2H, J=5Hz), 4.26, 4.55 (q, 2H, J=14Hz), 6.27 (s, 1H), 6.58 (s, 1H), 7.22 (s, 2H).

m.p.: 112 - 114°C (decomp.)

The inventive tablet was prepared in the same manner as that of Example 3 except for using Imidazole derivative H. The prepared tablet was subjected to the test for determining the remaining ratio of Imidazole derivative in acidic solutions as described in Example 3. It was found that the inventive tablet containing Imidazole derivative H gave high remaining ratios (similar to the ratios obtained in Example 3) in Solutions I and II.

EXAMPLE 11

A tablet of the following preparation was produced using Imidazole derivative A obtained in Example 3.

| Inventive Preparation (one tablet) | |
|---|---|
| Imidazole derivative A | 40.0 mg |
| Lactose | 50.0 mg |
| Crystalline cellulose (Avicel) | 20.0 mg |
| Carmethose calcium (ECG-505) | 15.0 mg |
| Magnesium hydroxide | 120.0 mg |
| Hydroxypropylcellulose (HPC-SL) | 3.0 mg |
| Magnesium stearate | 2.0 mg |
| Total | 250.0 mg |

Imidazole derivative A, lactose, crystalline cellulose, carmethose calcium and magnesium hydroxide were mixed and stirred. The mixture was then processed in the manner as in Example 3 to give the desired tablets.

The prepared tablet was subjected to the test for determining the remaining ratio of Imidazole derivative in acidic solutions as described in Example 3. It was found that the produced inventive tablet gave high remaining ratios (similar to the ratios obtained in Example 3) in Solutions I and II.

EXAMPLE 12

A following capsule preparation was produced using Imidazole derivative A obtained in Example 3.

| Inventive Preparation (one capsule) | |
|---|---|
| Imidazole derivative A | 40.0 mg |
| Lactose | 40.0 mg |
| Hydroxypropylcellulose (L-HPC) | 10.0 mg |
| Aluminum hydroxide gel | 200.0 mg |
| Hydroxypropylcellulose (HPC-SL) | 7.0 mg |
| Magnesium stearate | 3.0 mg |
| Total | 300.0 mg |

Imidazole derivative A, lactose, hydroxypropylcellulose (L-HPC), and aluminum hydroxide gel were mixed and stirred. The mixture was then processed in the manner as in Example 3 to give the desired mixture for capsule preparation to be dissolved in stomach.

The produced capsule was subjected to the test for determining the remaining ratio of Imidazole derivative in acidic solutions as described in Example 3. It was found that the produced inventive capsule gave high remaining ratios (similar to the ratios obtained in Example 3) in Solutions I and II.

**Claims**

**1.** An anti-ulcer composition to be dissolved in stomach to affect its walls which comprises 100 weight parts of an imidazole derivative having the formula:

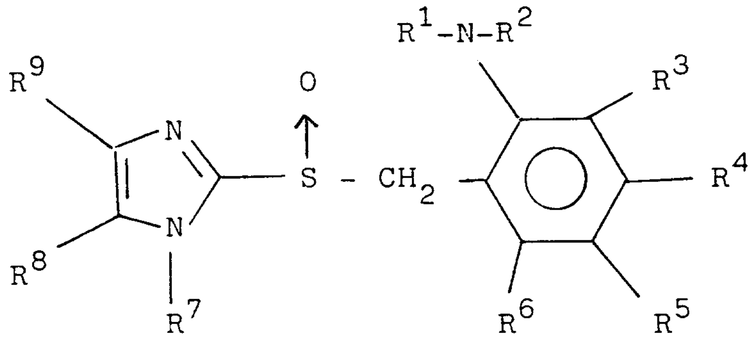

wherein:

each of $R^1$ and $R^2$ independently is hydrogen atom, an alkyl group having 1-8 carbon atoms, an alkyl group of 2-6 carbon atoms having an alkoxy group of 1-4 carbon atoms, a cycloalkyl group having 5-8 carbon atoms, an aryl group having 6-12 carbon atoms, an aralkyl group having 1-4 carbon atoms in its alkyl moiety and 6-12 carbon atoms in its aryl moiety, or an alkyl group of 1-8 carbon atoms having 1-3 halogen atoms, or $R^1$ and $R^2$ are combined to form, together with nitrogen atom to which $R^1$ and $R^2$ are attached, one of 5-8 membered heterocyclic rings;

each of $R^3$, $R^4$, $R^5$ and $R^6$ independently is hydrogen atom, a halogen atom, an alkoxy group having 1-6 carbon atoms, an alkyl group having 1-6 carbon atoms, an aralkyl group having 1-4 carbon atoms in its alkyl moiety and 6-12 carbon atoms in its aryl moiety, an aralkyloxy group having 1-4 carbon atoms in its alkoxy moiety and 6-12 carbon atoms in its aryl moiety, an alkoxycarbonyl group having 2-7 carbon atoms, nitro group, amino group, an acyl group having 1-7 carbon atoms, an alkyl group of 1-6 carbon atoms having 1-3 halogen atoms, or an alkoxy group of 1-6 carbon atoms having 1-3 halogen atoms, or $R^3$ is combined with $R^2$ to form, together with nitrogen atom to which $R^2$ is attached and two carbon atoms of benzene ring to which $R^3$ is attached, one of 5-8 membered heterocyclic rings;

$R^7$ is hydrogen atom, an alkyl group having 1-6 carbon atoms, an alkyl group of 1-6 carbon atoms which has at least one substituent selected from the group consisting of an aryl group of 4-12 carbon atoms, hydroxyl group, and a halogen atom, an aryl group having 4-12 carbon atoms, an aryl group of 4-12 carbon atoms which has at least one substituent selected from the group consisting of an alkyl group of 1-6 carbon atoms, an alkoxy group of 1-6 carbon atoms, and a halogen atom, an arylcarbonyl group having 7-13 carbon atoms, an arylcarbonyl group of 7-13 carbon atoms which has at least one substituent selected from the group consisting of an alkyl group of 1-6 carbon atoms, an alkoxy group of 1-6 carbon atoms, and a halogen atom, or a 5-8 membered heterocyclic group containing a sulfur atom as its ring member; and

each of $R^8$ and $R^9$ independently is hydrogen atom, a halogen atom, an alkoxy group having 1-6 carbon atoms, an alkyl group having 1-6 carbon atoms, an alkoxycarbonyl group having 2-7 carbon atoms, an aralkyl group having 1-4 carbon atoms in its alkyl moiety and 6-12 carbon atoms in its aryl moiety, nitro group, amino group, an acyl group having 1-7 carbon atoms, an alkyl group of 1-6 carbon atoms having 1-3 halogen atoms, an alkoxy group of 1-6 carbon atoms having 1-3 halogen atoms, an aryl group having 6-12 carbon atoms, or an aryl group of 6-12 carbon atoms which has at least one substitutent selected from the group consisting of an alkyl group of 1-6 carbon atoms, an alkoxy group of 1-6 carbon atoms and a halogen atom, or $R^8$ and $R^9$ are combined to form an alkylene chain of 3-5 carbon atoms,

and

50 to 2,000 weight parts of a basic material.

2. The anti-ulcer composition as defined in claim 1, wherein the basic material is comprised in an amount of 110 to 1,000 weight parts.

3. The anti-ulcer composition as defined in claim 1, wherein the basic material is comprised in an amount of 210 to 500 weight parts.

4. The anti-ulcer composition as defined in claim 1, wherein the basic material is selected from the group consisting of alumina magnesium hydroxide, aluminum hydroxide, magnesium hydroxide, magnesium carbonate, sodium hydrogen carbonate, calcium carbonate, sodium salt of a carboxylic acid, and potassium salt of a carboxylic acid.

5. The anti-ulcer composition as defined in claim 1, wherein each of $R^8$ and $R^9$ of the formula is hydrogen atom.

6. The anti-ulcer composition as defined in claim 1, wherein each of $R^1$ and $R^2$ of the formula independently is hydrogen atom, an alkyl group of 1-8 carbon atoms, or an alkyl group of 2-6 carbon atoms having an alkoxy group of 1-4 carbon atoms.

7. The anti-ulcer composition as defined in claim 1, wherein each of $R^3$, $R^4$ and $R^6$ of the formula is hydrogen atom, and $R^5$ is hydrogen atom or an alkoxy group of 1-6 carbon atoms.

8. The anti-ulcer composition as defined in claim 1, wherein $R^7$ of the formula is hydrogen atom.

European Patent Office

# EUROPEAN SEARCH REPORT

Application Number

EP 93 11 2415
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | CHEM. PHARM. BULL. vol. 39, no. 7, 1991, pages 1746 - 1752 TOMIO YAMAKAWA ET AL. 'Synthesis and structure-activity relationships of N-substituted 2-((imidazolylsulfinyl)methyl)anilines as a new class of gastric H+/K+-ATPase inhibitors' * page 1749, left column; table IV * * abstract * --- | 1-8 | A61K47/02<br>A61K47/08<br>A61K31/415 |
| D,Y | CHEM. PHARM. BULL. vol. 40, no. 3, 1992, pages 675 - 682 TOMIO YAMAKAWA ET AL. 'Synthesis and structure-activity relationships of substituted 2-((2-imidazolylsulfinyl)methyl)anilines as a new class of gastric H+/K+-ATPase inhibitors. II' * abstract; tables II,III * * page 680, left column * --- | 1-8 | TECHNICAL FIELDS SEARCHED (Int. Cl.5 )<br>A61K |
| D,Y | EP-A-0 248 634 (NIPPON CHEMIPHAR CO., LTD) * page 3, line 48 - page 6, line 19 * --- | 1-8 | |
| Y | EP-A-0 237 200 (TAKEDA CHEMICAL INDUSTRIES, LTD.) * page 2, line 16 - page 3, line 47 * * page 8, line 1 - line 58 * --- -/-- | 1-8 | |

The present search report has been drawn up for all claims

| Place of search | Date of completion of the search | Examiner |
|---|---|---|
| MUNICH | 26 NOVEMBER 1993 | TZSCHOPPE D. A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 93 11 2415
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | SCANDINAVIAN JOURNAL OF GASTROENTEROLOGY vol. 20, no. S108, 1985, pages 113 - 120 A. PILBRANDT ET AL. 'Development of an oral formulation of omeprazole' * page 113 * * page 116 * ----- | 1-8 | |

TECHNICAL FIELDS SEARCHED (Int. Cl.5 )

The present search report has been drawn up for all claims

| Place of search | Date of completion of the search | Examiner |
|---|---|---|
| MUNICH | 26 NOVEMBER 1993 | TZSCHOPPE D. A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document